(19) Europäisches Patentamt
European Patent Office
Office européen des brevets

(11) Veröffentlichungsnummer: **0 046 189**
**B1**

(12) **EUROPÄISCHE PATENTSCHRIFT**

(45) Veröffentlichungstag der Patentschrift: **04.01.84**

(51) Int. Cl.³: **C 07 D 498/08** // (C07D498/08, 261/00, 231/00)

(21) Anmeldenummer: **81105446.9**

(22) Anmeldetag: **13.07.81**

(54) **Isoxazolino-azoxi-verbindungen und Verfahren zu ihrer Herstellung.**

(30) Priorität: **20.08.80 DE 3031337**

(43) Veröffentlichungstag der Anmeldung: **24.02.82 Patentblatt 82/8**

(45) Bekanntmachung des Hinweises auf die Patenterteilung: **04.01.84 Patentblatt 84/1**

(84) Benannte Vertragsstaaten: **BE CH DE FR GB LI**

(56) Entgegenhaltungen:
**HOUBEN-WEYL, "Methoden der organischen Chemie", 4. Auflage, Band X/2, 21. September 1967, Stuttgart "Herstellung aliphatischer Azoxyverbindungen", GEROG THIEME VERLAG Seite 787**

(73) Patentinhaber: **BASF Aktiengesellschaft, Carl-Bosch-Strasse 38, D-6700 Ludwigshafen (DE)**

(72) Erfinder: **Rieber, Norbert, Dr., Liebfrauenstrasse 1c, D-6800 Mannheim (DE)**
Erfinder: **Boehm, Heinrich, Dr., Keplerweg 2, D-6701 Neuhofen (DE)**
Erfinder: **Platz, Rolf, Dr., Hansastrasse 5, D-6800 Mannheim (DE)**
Erfinder: **Fuchs, Werner, Dr., Muenchbuschweg 30B, D-6700 Ludwigshafen (DE)**

**EP 0 046 189 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Isoxazolinoazoxiverbindungen und Verfahren zu ihrer Herstellung

Die Erfindung betrifft Isoxazolinoazoxiverbindungen und Verfahren zu ihrer Herstellung durch Umsetzung von Isoxazolinoazoverbindungen mit organischen Peroxiden.

Es ist aus Houben-Weyl, „Methoden der organischen Chemie", Bd. X/2, S. 787, bekannt, dass man Azocyclododecan in Gegenwart von Natriumacetat und Essigsäureäthylester mit Peressigsäure in Azocyclododecan umwandeln kann. Persäuren wirken hingegen auf β-Aminopropionsäurenitril in der Weise ein, dass 4 Molekeln Nitril kondensieren, wobei eine Azoxigruppe, 2 Carbonamidogruppen und 2 Carbonimidogruppen im Kondensationsprodukt gebildet werden. Ebenso ist aus Houben-Weyl *(loc. cit.)*, Bd. 8, S. 68 bis 70, bekannt, dass Persäuren sowohl Sauerstoffverbindungen oxidieren, wie auch an tertiäre Stickstoffatome ein Sauerstoffatom lagern, wobei Aminoxide gebildet werden.

Es wurde nun gefunden, dass man Isoxazolinoazoxiverbindungen der Formel

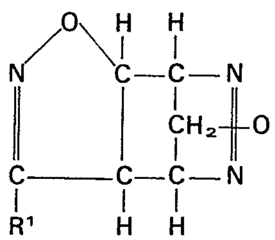

(I)

worin $R^1$ ein Wasserstoffatom, einen gegebenenfalls durch eine Hydroxygruppe substituierten Alkylrest mit 1 bis 18 Kohlenstoffatomen, einen Cycloalkylrest mit 1 bis 18 Kohlenstoffatomen, einen Cycloalkylrest mit 3 bis 8 Kohlenstoffatomen, einen Aralkylrest mit 7 bis 12 Kohlenstoffatomen oder einen unsubstituierten oder durch Bromatome, Fluoratome, Chloratome, Nitrogruppen, Trifluormethyl, Hydroxygruppen, Alkoxygruppen und/oder durch Fluor substituierte Alkoxygruppen mit jeweils 1 bis 4 Kohlenstoffatomen je Alkylgruppe substituierten Phenylrest oder Naphthylrest bedeutet, vorteilhaft erhält, wenn man Isoxazolinoazoverbindungen der Formel

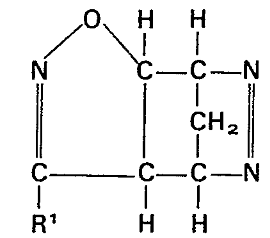

(II)

worin $R^1$ die vorgenannte Bedeutung besitzt, mit einem organischen Peroxid umsetzt.

Weiterhin wurden die neuen Isoxazolinoazoxiverbindungen der Formel

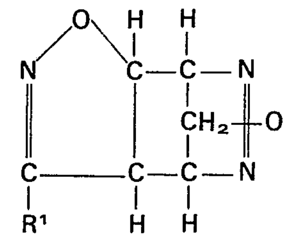

(I)

worin $R^1$ ein Wasserstoffatom, einen aliphatischen, cycloaliphatischen, araliphatischen oder aromatischen Rest bedeutet, die wie vorher definiert sind, gefunden.

Die Umsetzung kann für den Fall der Verwendung von 2,3,7-Triaza-6-oxatricyclo-$[5,2,1,0^{5,9}]$-deca-2,7-dien und Peressigsäure durch die folgenden Formeln wiedergegeben werden:

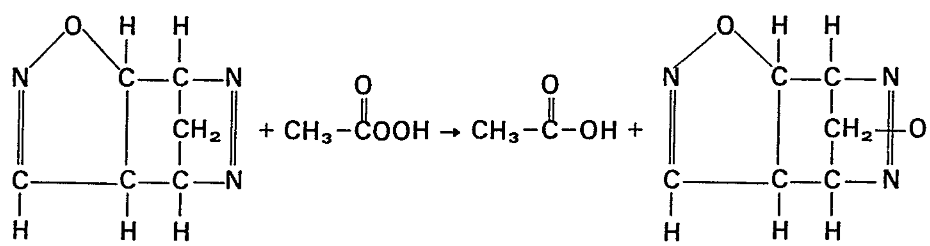

Das Verfahren nach der Erfindung liefert auf einfachem und wirtschaftlichem Wege die neuen Isoxazolinoazoxiverbindungen in guter Ausbeute und Reinheit. Alle diese vorteilhaften Ergebnisse sind mit Bezug auf den Stand der Technik überraschend.

Die Ausgangsstoffe II können leicht durch Umsetzung in einem ersten Schritt von Nitriloxiden der Formel

$$R^1CNO \quad (III)$$

worin $R^1$ die vorgenannte Bedeutung besitzt, mit N,N'-Dicarbalkoxy-2,3-diazabicyclo-[2,2,1]-hept-2-enen der Formel

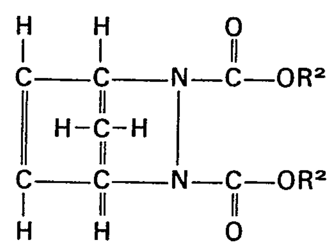

(IV)

worin die einzelnen Reste $R^2$ gleich oder verschieden sein können und jeweils ein Wasserstoffatom oder einen aliphatischen Rest bedeuten, in die Stoffe V der Formel

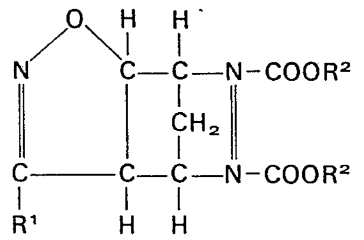 (V)

worin $R^1$ und $R^2$ die vorgenannten Bedeutungen besitzen, und dann in einem 2. Schritt die Stoffe V durch Oxidation in Gegenwart von basischen Verbindungen hergestellt werden.

Die Ausgangsstoffe III sind leicht durch die in Houben-Weyl, „Methoden der organischen Chemie", Bd. 10/3, S. 841 bis 853 beschriebenen Arbeitsweise, z.B. durch Dehydrierung von Aldoximen oder aus Hydroxamsäurederivaten oder Nitrolsäuren zugänglich. Die Ausgangsstoffe IV erhält man z.B. durch Umsetzung von Cyclopentadien mit Azodicarbonsäuredialkylester nach den in „Ann." 443, 249 bis 262 (1925) beschriebenen Verfahren.

Bevorzugte Ausgangsstoffe II und dementsprechend bevorzugte Stoffe III, IV, V und Endstoffe I sind solche, in deren Formeln die einzelnen Reste $R^1$ und $R^2$ gleich oder verschieden sein können und jeweils ein Wasserstoffatom oder einen gegebenenfalls durch eine Hydroxygruppe substituierten Alkylrest mit 1 bis 18, vorteilhaft 1 bis 12, insbesondere 1 bis 4 Kohlenstoffatomen bedeuten, $R^1$ auch einen Cycloalkylrest mit 3 bis 8 Kohlenstoffatomen, einen Aralkylrest mit 7 bis 12 Kohlenstoffatomen oder einen unsubstituierten oder durch 1, 2 oder 3 Bromatome, Fluoratome, Chloratome, Nitrogruppen, Trifluormethyl, Hydroxygruppen, Dialkylaminogruppen, Alkoxygruppen und/oder durch Fluor substituierte Alkoxygruppen mit jeweils 1 bis 4 Kohlenstoffatomen je Alkylgruppe substituierten Phenylrest oder Naphthylrest bezeichnet.

Alle Stoffe III werden zweckmässig im ersten Verfahrensschritt *in situ* aus ihren Vorprodukten, z.B. aus den Oximen der Formel

$$R^1-CH=N-OH \qquad (VI)$$

durch Dehydrierung oder den Hydroxamsäurehalogeniden der Formel

$$R^1-\underset{\displaystyle X}{\underset{|}{C}}=N-OH \qquad (VII)$$

worin R die vorgenannte Bedeutung besitzt und X ein Halogenatom, vorzugsweise ein Bromatom oder Chloratom, bezeichnet, durch Halogenwasserstoffabspaltung unter Verwendung basischer Stoffe hergestellt.

Es kommen z.B. als Ausgangsstoffe III in Betracht: Methyl-, Äthyl-, Propyl-, Isopropyl-, Butyl-, sek.-Butyl-, tert.-Butyl-, Isobutyl-, Pentyl-, Hexyl-, Heptyl-, Octyl-, Nonyl-, Decyl-, Undecyl-, Dodecyl-, Cyclopropyl-, Cyclohexyl-, Cyclopentyl-, Benzyl-, Phenyl, 2-, 3- oder 4-Chlorphenyl-, 2-, 3- oder 4-Bromphenyl-, 2-, 3-, 4-Methoxyphenyl, o-, m-, p-Methylphenylnitriloxid.

Als Ausgangsstoffe IV kommen in Frage: N,N'-Dicarbmethoxy-2,3-diazabicyclo-[2,2,1]-hept-2-en; Homologe Diäthyl-, Dipropyl-, Diisopropyl-, Dibutyl-, Diisobutyl-, Disek.-butyl-, Ditert.-butylester vorgenannter Heptene.

Die Umsetzung der Ausgangsstoffe III und IV wird vorteilhaft in der ersten Stufe bei einer Temperatur von −25 bis 100, vorzugsweise von 0 bis 40°C, drucklos oder unter Druck, kontinuierlich oder diskontinuierlich durchgeführt. Zweckmässig verwendet man unter den Reaktionsbedingungen inerte Lösungsmittel. Als Lösungsmittel kommen z.B. in Frage: Halogenkohlenwasserstoffe, insbesondere Brom- und Chlorkohlenwasserstoffe, z.B. Tetrachloräthylen, 1,1,2,2- oder 1,1,1,2-Tetrachloräthan, Amylchlorid, Cyclohexylchlorid, 1,2-Dichlorpropan, Methylenchlorid, Dichlorbutan, Chlornaphthalin, Dichlornaphthalin, Tetrachlorkohlenstoff, 1,1,1- oder 1,1,2-Trichloräthan, Trichloräthylen, Pentachloräthan, 1,2-Dichloräthan, 1,1-Dichloräthan, 1,2-cis-Dichloräthan, 1,2-cis-Dichloräthylen, Chlorbenzol, Fluorbenzol, Brombenzol, Jodbenzol, o-, p- und m-Dichlorbenzol, o-, p-, m-Dibrombenzol, o-, m-, p-Chlortoluol, 1,2,4-Trichlorbenzol, 1,10-Dibromdekan, 1,4-Dibrombutan; Äther, z.B. Äthylpropyläther, Methyl-tert.-butyläther, n-Butyläthyläther, Di-n-butyläther, Diisobutyläther, Diisoamyläther, Diisopropyläther, Anisol, Phenetol, Cyclohexylmethyläther, Diäthyläther, Äthylenglykoldimethyläther, Tetrahydrofuran, Dioxan, Thioanisol, β,β'-Dichlordiäthyläther, und entsprechende Gemische. Zweckmässig verwendet man das Lösungsmittel in einer Menge von 100 bis 10 000, vorzugsweise von 100 bis 2000 Gew.-%, bezogen auf Ausgangsstoff III.

Die Reaktion des ersten Schrittes kann wie folgt durchgeführt werden: Ein Gemisch der Ausgangsstoffe III und IV und zweckmässig Lösungsmittel wird während 3 bis 22 h bei der Reaktionstemperatur gehalten. Der Ausgangsstoff III wird *in situ* hergestellt, indem man z.B. dem zugrundeliegenden Oxim ein Oxidationsmittel, z.B. Natriumhypochloritlösung, oder z.B. dem zugrundeliegenden Hydroxamsäurechlorid ein tertiäres Amin, z.B. Triäthylamin, zugibt. Dann wird der Stoff V aus dem Gemisch nach bekannten Methoden, z.B. durch Filtration oder Extraktion und fraktionierte Destillation, abgetrennt.

Der 2. Schritt der Umsetzung der Stoffe III und IV wird mit einer basischen Verbindung, vorteilhaft in einer Menge von 4 bis 25, vorzugsweise von 4 bis 8 Äquivalenten basischer Verbindung, bezogen auf 1 mol Ausgangsstoff III, durchgeführt. Bevorzugte basische Verbindungen sind Erdalkaliverbindungen, Ammoniumverbindungen und insbesondere Alkaliverbindungen, vorteilhaft wässerige oder alkoholische Natron- oder Kalilauge, sowie entsprechende Gemische. Es kommen z.B. als basische Verbindungen in Frage: Kaliumhydroxid, Natriumhydroxid, Kaliumcarbonat, Lithiumhydroxid, Lithiumcarbonat, Natriumbicarbonat, Kaliumbicarbonat, Calciumhydroxid, Calciumoxid, Bariumoxid, Magnesiumhydroxid, Magnesiumoxid, Bariumhydroxid, Ammoniumhydroxid.

Der 2. Schritt der Umsetzung der Stoffe III und IV wird mit einem Oxidationsmittel, vorzugsweise Hypohalogeniten oder Wasserstoffperoxid, durchgeführt. Das Wasserstoffperoxid wird zweckmässig in Form seiner 1- bis 90-, vorzugsweise 20- bis 55gew.-igen, wässerigen Lösung verwendet. Gegebenenfalls kommen auch Stoffe in Betracht, die unter den Umsetzungsbedingungen Wasserstoffperoxid bilden, z.B. anorganische oder organische Peroxoverbindungen wie Natriumperoxid, Kaliumperoxid, Magnesiumperoxid, Calciumperoxid, Zinkperoxid, Bariumperoxid, Bariumsuperoxid; Hydroperoxide wie $NaOOH \cdot 0{,}5\ H_2O_2$, $NH_4OOH$; entsprechende Hydrate wie $CaO_2 \cdot 8H_2O$, Peroxohydrate wie $BaO_2 \cdot H_2O_2$ und $BaO_2 \cdot 2\ H_2O_2$; Peroxocarbonate wie Natriumperoxocarbonat und Calciumperoxocarbonat; Peroxophosphate wie das Kaliumperoxodiphosphat. Ebenfalls können Wasserstoffperoxidanlagerungsverbindungen verwendet werden wie Natriumboratperoxohydrat, Natriumcarbonatperoxohydrat. Gegebenenfalls können Hilfsstoffe wie Magnesiumsulfat oder Magnesiumchlorid zugesetzt werden.

Als Oxidationsmittel verwendet man vorteilhaft im 2. Schritt der Umsetzung der Stoffe III und IV auch Hypohalogenite, in der Regel Hypochlorite und Hypobromite in wässerigem Medium, in der Regel in Gestalt von entsprechenden wässerigen, alkalischen Lösungen. Die wässerigen Hypohalogenitlösungen, vorzugsweise Hypochloritlösungen, enthalten im allgemeinen von 5 bis 25, vorzugsweise 12 bis 14 Gew.-% Hypohalogenit, vorzugsweise Hypochlorit, und können zweckmässig zusätzlich von 0,2 bis 2,5 mol, vorzugsweise 0,5 bis 2,1 mol Alkalihydroxid/mol Hypohalogenit, vorzugsweise Hypochlorit, enthalten. Im Ausgangsgemisch beider Ausgangsstoffe kommen im allgemeinen Mengen von 0,9 bis 2,5, vorzugsweise 0,95 bis 1,1 mol Hypochlorit und zweckmässig von insgesamt 4 bis 25 mol, vorzugsweise 4 bis 8 mol Alkalihydroxid (nicht eingerechnet das im Hypohalogenit enthaltene Alkali), bezogen auf 1 mol Ausgangsstoff III, in Frage. Bevorzugte Alkalihypohalogenite sind das Natrium- oder das Kaliumsalz.

Die Umsetzung des Stoffs V im 2. Schritt wird in der Regel bei einer Temperatur von 20 bis 180, vorzugsweise 40 bis 120°C, drucklos oder unter Druck, kontinuierlich oder diskontinuierlich durchgeführt. Zweckmässig verwendet man unter den Reaktionsbedingungen inerte Lösungsmittel. Als Lösungsmittel kommen z.B. in Frage: Wasser; Alkanole und Cycloalkanole wie Äthanol, Methanol, n-Butanol, Isobutanol, tert.-Butanol, Glykol, Glycerin, n-Propanol, Isopropanol, Amylalkohol, Cyclohexanol, und entsprechende Gemische. Zweckmässig verwendet man das Lösungsmittel in einer Menge von 100 bis 10 000, vorzugsweise von 100 bis 1000 Gew.-%, bezogen auf Ausgangsstoff III.

Die Reaktion des 2. Schrittes kann wie folgt durchgeführt werden: Ein Gemisch von Stoff V, basischer Verbindung, Oxidationsmittel und zweckmässig Lösungsmittel, wird während 2 bis 8 h bei der Reaktionstemperatur des 2. Schrittes gehalten. In der Regel wird man zunächst Stoff V, basische Verbindung und Lösungsmittel einige Zeit, zweckmässig 1 bis 5 h, bei der Reaktionstemperatur des 2. Schrittes halten, dann das Oxidationsmittel, zweckmässig zusammen mit einem weiteren Anteil an Lösungsmittel, zusetzen und die Reaktion, zweckmässig während 1 bis 7 h, durchführen. Aus dem Reaktionsgemisch wird der Ausgangsstoff II in üblicher Weise, z.B. durch Filtration, Extraktion, Einengen des Filtrats und Filtration, abgetrennt.

Die folgenden Ausgangsstoffe II kommen beispielsweise in Frage: 2,3,7-Triaza-6-oxatricyclo-$[5,2,1,0^{5,9}]$-deca-2,7β-dien und seine in 8-Stellung durch die Methyl-, Äthyl-, Propyl-, Isopropyl-, Butyl-, Isobutyl-, sek.-Butyl-, tert.-Butyl-, sek.-Heptyl-, Cyclohexyl-, Cyclopentyl-, Benzyl-, Phenyl-, Naphthyl-, 2-Chlorphenyl-, 3-Chlorphenyl-, 4-Chlorphenyl-, 2,4-Dichlorphenyl, 2-Methylphenyl-, 3-Methylphenyl-, 4-Methylphenyl-, 2-Methoxyphenyl-, 3-Methoxyphenyl-, 4-Methoxyphenyl-, 2-Äthylphenyl-, 3-Äthylphenyl-, 4-Äthylphenyl-, 2-Äthoxyphenyl-, 3-Äthoxyphenyl-, 4-Äthoxyphenylgruppe substituierten Homologen.

Die erfindungsgemässe Umsetzung der Ausgangsstoffe II wird in der Regel bei einer Temperatur von 10 bis 150, vorzugsweise 20 bis 100, insbesondere 50 bis 90°C, drucklos oder unter Druck, diskontinuierlich oder kontinuierlich durchgeführt. Man kann unter den Reaktionsbedingungen inerte, organische Lösungsmittel verwenden oder in Abwesenheit organischer Lösungsmittel umsetzen. Als Lösungsmittel kommen z.B. in Frage: Halogenkohlenwasserstoffe, insbesondere Brom- und Chlorkohlenwasserstoffe, z.B. Tetrachloräthylen, 1,1,2,2- oder 1,1,1,2-Tetrachloräthan, Amylchlorid, Cyclohexylchlorid, 1,2-Dichlorpropan, Methylenchlorid, Dichlorubtan, Chloroform, Chlornaphthalin, Dichlornaphthalin, Tetrachlorkohlenstoff, 1,1,1- oder 1,1,2-Trichloräthan, Trichloräthylen, Pentachloräthan, 1,2-Dichloräthan, 1,1-Dichloräthan, 1,2-cis-Dichloräthylen, Chlorbenzol, Fluorbenzol, Brombenzol, Jodbenzol, o-, p- und m-Dichlorbenzol, o-, p-, m-Dibrombenzol, o-, m-, p-Chlortoluol, 1,2,4-Trichlorbenzol, 1,10-Dibromdekan, 1,4-Dibrombutan; Äther, z.B. Äthylpropyläther, Methyltert.-Butyläther. n-Butyläthyläther, Di-n-butyläther, Diisobutyläther, Diisoamyläther, Diisopropyläther, Anisol, Phenetol, Cyclohexylmethyläther, Diäthyläther, Äthylenglykoldimethyläther, Tetrahydrofuran, Dioxan, β,β'-Dichlordiäthyläther, und entsprechende Gemische. Zweckmässig verwendet man das Lösungsmittel in einer Menge von 20 bis 10 000, vorzugsweise von 30 bis 1000 Gew.-%, bezogen auf Ausgangsstoff II.

Als weiteren Ausgangsstoff werden organische Peroxide, zweckmässig Alkylhydroperoxide, Dialkylperoxide, Hydroperoxidester, Diacylperoxide, Peroxidderivate von Aldehyden und Ketonen, bevorzugt aber Persäuren verwendet. Es kommen im allgemeinen Peroxide der Formel

$$R^4{-}OOH \qquad (VIII)$$

und

$$R^4{-}O{-}O{-}R^4 \qquad (IX)$$

worin die einzelnen Reste $R^4$ gleich oder verschieden sein können und jeweils den Rest $R^3$–,

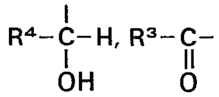

bedeuten, die 2 Reste $R^4$ der Formel IX zusammen auch den Rest

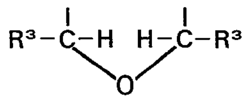

oder den Rest $-R^5-$ bezeichnen, $R^3$ und $R^4$ für einen aliphatischen, cycloaliphatischen, araliphatischen oder aromatischen Rest, vorteilhaft $R^3$ für einen Alkylrest mit 1 bis 12 Kohlenstoffatomen, einen Cycloalkylrest mit 5 bis 8 Kohlenstoffatomen, einen Aralkylrest oder Alkylarylrest mit 7 bis 16 Kohlenstoffatomen, einen gegebenenfalls durch Nitrogruppen, Bromatome, Chloratome substituierten Phenylrest, $R^5$ vorteilhaft für einen Alkylenrest mit 2 bis 6 Kohlenstoffatomen stehen. Die vorgenannten Reste können noch durch unter den Reaktionsbedingungen inerte Gruppen, z.B. Nitrogruppen, Alkylgruppen, Alkoxygruppen mit jeweils 1 bis 4 Kohlenstoffatomen substituiert sein. Schon aus Gründen der besseren Handhabung wird man die weniger explosionsgefährdeten höhermolekularen Peroxide oder Persäuren in Betracht ziehen. Bezüglich der Auswahl wird in diesem Zusammenhang auf Houben-Weyl *(loc. cit.)*, Bd. 8, S. 6 bis 9, bezüglich der Herstellung und Handhabung von Peroxiden auf die Seiten 6 bis 72 verwiesen.

Es kommen z.B. als Peroxide in Frage: Isopropylhydroperoxid, tert.-Butylhydroperoxid, Triphenylmethylhydroperoxid, Bekalinhydroperoxid; Diäthylperoxid, Di-tert.-butylperoxid, polymeres Peroxid des asymetrischen Diphenyläthylens, Peroxid des Dimethylketens, Photoxid des Anthracens; p-Nitrobenzoesäureester von tertiären Hydroxiden, 1,1-Bisbenzoperoxycyclohexan, 1,1-Bis-p-nitrobenzoperoxycyclohexan; Dicapronylperoxid, Dibenzoylperoxid, α-Naphthoylperoxid, Oxymethylhydroperoxid, Oxyäthyläthylperoxid, Dioxydicyclohexylperoxid, Bishydroperoxydicyclohexylperoxid, 1-Hydroperoxy-1-acetoxycyclodecanon-6, 2,2-Bis(tert.-butylperoxy)butan, Butylenozonid, Ozonid des 1,2-Dimethylcyclopentens. Bevorzugt sind Perameisensäure, Peressigsäure, Perbenzoesäure, Percapronsäure, 2-Chlorperbenzoesäure, 3-Chlorperbenzoesäure, 4-Chlorperbenzoesäure. Man kann mit dem Peroxid in stöchiometrischer Menge oder im Überschuss, bevorzugt in einem Verhältnis von 1 bis 10, insbesondere 1 bis 1,5 mol Peroxid/mol Ausgangsstoff II, umsetzen. Man kann das Peroxid auch vorteilhaft aus Stoffen, die unter den Reaktionsbedingungen solche Peroxide bilden, *in situ* herstellen, z.B. anstelle von Peressigsäure Essigsäureanhydrid und wässerige $H_2O_2$-Lösung verwenden.

Die Reaktion kann wie folgt durchgeführt werden: Ausgangsstoff II, Peroxid und gegebenenfalls Lösungsmittel wird während 1 bis 48 h bei der Reaktionstemperatur gehalten. Dann wird der Endstoff aus dem Reaktionsgemisch in üblicher Weise, z.B. durch Filtration und fraktionierte Destillation, Extraktion oder Kristallisation, abgetrennt.

Die nach dem Verfahren der Erfindung erhältlichen Isoxazolinoazoxiverbindungen I sind wertvolle Ausgangsstoffe für die Herstellung von Farbstoffen, Schädlingsbekämpfungsmitteln und Pharmazeutika.

Sie sind wertvolle UV-Schutzmittel. Sie absorbieren in dem für die Bräunung der Hautschädlichen UV-B- und C-Bereich (218 bis 315 nm) der natürlichen UV-Strahlung. Der Absorptionsbereich der Verbindungen liegt zwischen 220 und 300 nm. Es wird bezüglich allgemeinen Eigenschaften und Verwendung von Lichtschutzmitteln auf die folgenden Veröffentlichungen verwiesen: „Naturwiss. Rdsch." *25*, 89 bis 99 (1972). Ebenfalls können aus den Endstoffen I auch anderartig substituierte Isoazoline hergestellt werden. 1-Hydroxypyrazol kann durch Umsetzung der Isoxazolinoazoxiverbindungen I bei 140 bis 600°C hergestellt werden. 1-Hydroxypyrazol ist bisher nur in Form von Derivaten mit mehreren Substituenten an den C-Atomen des Pyrazolringes bekannt. So wird in „J. org. Chem.", Bd. 34, S. 194 bis 198 (1969), die Synthese von 3fach substituierten 1-Hydropyrazolen auf folgendem Weg beschrieben:

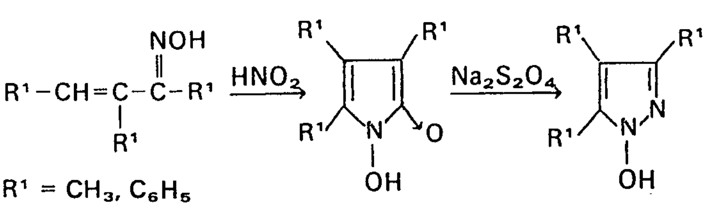


$R^1 = CH_3, C_6H_5$

1-Hydroxypyrazol kann leicht analog der in „J. org. Chem.", *loc. cit.*, beschriebenen Arbeitsweise durch Reduktion mit Zink in das entsprechende Pyrazol überführt werden. Bezüglich der Verwendung wird auf die vorgenannten Veröffentlichungen und „Ullmanns Encyklopädie der technischen

Chemie", Bd. 8, S. 498 und 500, Bd. 18, S. 548, verwiesen.

Die in den folgenden Beispielen angeführten Teile bedeuten Gewichtsteile.

*Beispiel 1*

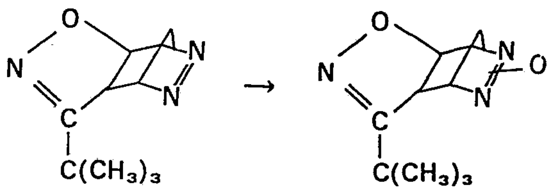

70 Teile 8-t-Butyl-2,3,7-triaza-6-oxatricyclo-[5,2,1,$0^{5,9}$]-deca-2,7-dien und 125 Teile 3-Chlorperbenzoesäure in 800 Teilen 1,1,2-Trichloräthan werden 12 h bei 70°C gerührt. Nach dem Abkühlen auf 25°C wird die ausgefallene 3-Chlorbenzoesäure abfiltriert und der Rückstand mit 50 Teilen 1,1,2-Trichloräthan gewaschen. Die vereinigten Filtrate werden nacheinander mit verdünnten wässerigen Lösungen von Kaliumjodid, Natriumthiosulfat und Natriumhydrogencarbonat extrahiert und im Rotationsverdampfer bei 15 mbar und 40°C eingeengt. Man erhält 69 Teile (91% der Theorie) 8-t-Butyl-2,3-azoxi-7-aza-6-oxatricyclo-[5,2,1,$0^{5,9}$]-deca-2,7-dien vom Fp. 129°C (Ligroin/Toluol).

*Beispiel 2*

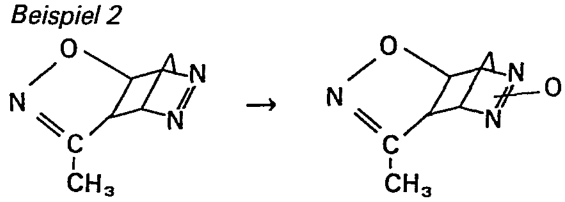

Zu 25 Teilen 8-Methyl-2,3,7-triaza-6-oxatricyclo-[5,2,1,$0^{5,9}$]-deca-2,7-dien in 33 Teilen Essigsäureanhydrid gibt man bei 70°C unter Rühren 35 Teile 50gew.-%ige, wässerige $H_2O_2$-Lösung zu und rührt das Gemisch noch 3 h bei 70°C. Nach Abkühlen auf 0°C wird der ausgefallene Feststoff abgesaugt und mit 5 Teilen Äther gewaschen. Das Filtrat wird mit 3×50 Teilen $CH_2Cl_2$ extrahiert. Die $CH_2Cl_2$-Phase wird mit 20 Teilen 25gew.-%iger, wässeriger Natronlauge und mit 20 Teilen Wasser gewaschen und anschliessend im Rotationsverdampfer bei 15 mbar und 40°C eingeengt. Der Filtratrückstand und der Rückstand aus der $CH_2Cl_2$-Phase werden vereinigt. Man erhält 25 Teile (90% der Theorie) 8-Methyl-2,3-azoxi-7-aza-6-oxatricyclo-[5,2,1,$0^{5,9}$]-deca-2,7-dien vom Fp. 126°C.

*Beispiele 3 bis 15*

Entsprechend den Beispielen 1 und 2 in Reaktionsparametern und Molverhältnissen der Reaktionskomponenten werden die in der Tabelle aufgeführten Endstoffe hergestellt.

*Tabelle*

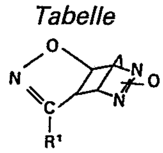

| Beispiel | $R^1$ | Umsetzung analog Beispiel | Teile Ausgangsstoff II | Endstoff Ausbeute in % der Theorie | Fp. (°C) |
|---|---|---|---|---|---|
| 3 | $CH_3-CH_2-$ | 2 | 20 | 93 | 173 |
| 4 | $CH_3-(CH_2)_2-$ | 2 | 25 | 89 | 117 |
| 5 | $CH_3-CH-$<br>$CH_3$ | 2 | 25 | 85 | 49 |
| 6 | $HO-CH_2-C(CH_3)_2-$ | 1 | 15 | 79 | 77 |
| 7 | $CH_3-(CH_2)_3-CH-$<br>$C_2H_5$ | 2 | 20 | 90 | 72 |
| 8 | 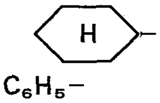 | 2 | 25 | 91 | 150 |
| 9 | $C_6H_5-$ | 1 | 20 | 88 | 130 |
| 10 | $p-Cl-C_6H_4-$ | 2 | 25 | 89 | 157 |
| 11 | $C_6H_5-CH_2-$ | 2 | 10 | 78 | 109 |
| 12 | $p-CH_3-C_6H_4$ | 2 | 10 | 86 | 143 |
| 13 | $p-CH_3O-C_6H_4-$ | 2 | 15 | 92 | 168 |
| 14 | 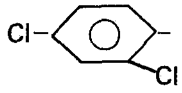 | 2 | 15 | 84 | 152 |
| 15 | 1-Naphthyl | 2 | 10 | 82 | 125 |

## Patentansprüche

1. Isoxazolinoazoxiverbindungen der Formel

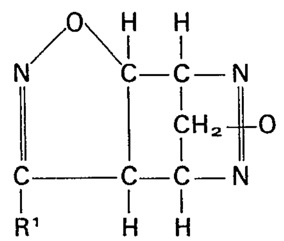

(I)

worin $R^1$ ein Wasserstoffatom, einen gegebenenfalls durch eine Hydroxygruppe substituierten Alkylrest mit 1 bis 18 Kohlenstoffatomen, einen Cycloalkylrest mit 3 bis 8 Kohlenstoffatomen, einen Aralkylrest mit 7 bis 12 Kohlenstoffatomen oder einen unsubstituierten oder durch Bromatome, Fluoratome, Chloratome, Nitrogruppen, Trifluormethyl, Hydroxygruppen, Alkoxygruppen und/oder durch Fluor substituierte Alkoxygruppen mit jeweils 1 bis 4 Kohlenstoffatomen je Alkylgruppe substituierten Phenylrest oder Naphthylrest bedeutet.

2. Verfahren zur Herstellung von Isoxazolinoazoxiverbindungen der Formel (I) gemäss Anspruch 1

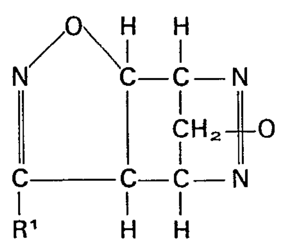

(I)

worin $R^1$ ein Wasserstoffatom, einen gegebenenfalls durch eine Hydroxygruppe substituierten Alkylrest mit 1 bis 18 Kohlenstoffatomen, einen Cycloalkylrest mit 3 bis 8 Kohlenstoffatomen, einen Aralkylrest mit 7 bis 12 Kohlenstoffatomen oder einen unsubstituierten oder durch Bromatome, Fluoratome, Chloratome, Nitrogruppen, Trifluormethyl, Hydroxygruppen, Alkoxygruppen und/oder durch Fluor substituierte Alkoxygruppen mit jeweils 1 bis 4 Kohlenstoffatomen je Alkylgruppe substituierten Phenylrest oder Naphthylrest bedeutet, dadurch gekennzeichnet, dass man Isoxazolinoazoverbindungen der Formel

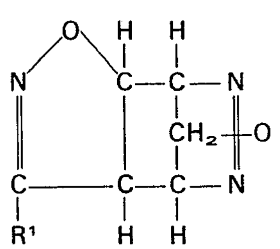

(I)

worin $R^1$ die vorgenannte Bedeutung besitzt, mit einem organischen Peroxid umsetzt.

## Revendications

1. Composés isoxazolinoazoxyliques de formule

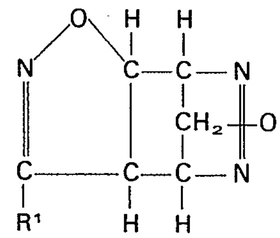

(I)

dans laquelle $R^1$ représente un atome d'hydrogène, un groupe alkyle en $C_1$-$C_{18}$, éventuellement substitué par un groupe hydroxy, un groupe cycloalkyle en $C_3$-$C_8$, un groupe aralkyle en $C_7$-$C_{12}$ ou un groupe phényle ou naphtyle non substitué ou substitué par des atomes de brome, des atomes de fluor, des atomes de chlore, des groupes nitro, trifluorométhyle, des groupes hydroxy, des groupes alcoxy et/ou par des groupes alcoxy substitués par le fluor et contenant chacun de 1 à 4 atomes de carbone par groupe alkyle.

2. Procédé de préparation des composés isoxazolinoazoxyliques de formule (I) selon la revendication 1

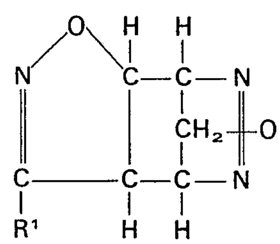

(I)

dans laquelle $R^1$ représente un atome d'hydrogène, un groupe alkyle en $C_1$-$C_{18}$, éventuellement substitué par un groupe hydroxy, un groupe cycloalkyle en $C_3$-$C_8$, un groupe aralkyle en $C_7$-$C_{12}$ ou un groupe phényle ou naphtyle non substitué ou substitué par des atomes de brome, des atomes de fluor, des atomes de chlore, des groupes nitro, trifluorométhyle, des groupes hydroxy, des groupes alcoxy et/ou des groupes alcoxy substitués par le fluor et contenant chacun 1 à 4 atomes de carbone par groupe alkyle, caractérisé en ce que l'on fait réagir des composés isoxazolinoazoïques de formule

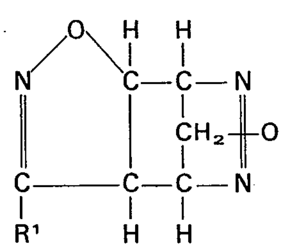

(I)

dans laquelle $R^1$ a la signification indiquée ci-dessus, avec un peroxyde organique.

## Claims

1. An isoxazolinoazoxy compound of the formula

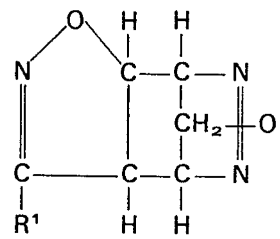

(I)

where $R^1$ is hydrogen, alkyl of 1 to 18 carbon atoms optionally substituted by a hydroxyl group, cycloalkyl of 3 to 8 carbon atoms, aralkyl of 7 to 12 carbon atoms, unsubstituted phenyl or naphthyl, or phenyl or naphthyl substituted by bromine, fluorine or chlorine atoms, nitro groups, trifluoromethyl, hydroxyl groups, alkoxy groups and/or by fluorine-substituted alkoxy groups where alkyl is in each case of 1 to 4 carbon atoms.

2. A process for the preparation of an isoxazolinoazoxy compound of the formula (I) as claimed in claim 1

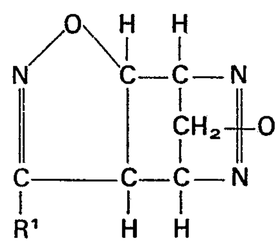

(I)

where $R^1$ is hydrogen, alkyl of 1 to 18 carbon atoms optionally substituted by a hydroxyl group, cycloalkyl of 3 to 8 carbon atoms, aralkyl of 7 to 12 carbon atoms, unsubstituted phenyl or naphthyl, or phenyl or naphthyl substituted by bromine, fluorine or chlorine atoms, nitro groups, trifluoromethyl, hydroxyl groups, alkoxy groups and/or by fluorine-substituted alkoxy groups where alkyl is in each case of 1 to 4 carbon atoms, wherein an isoxazolinoazo compound of the formula

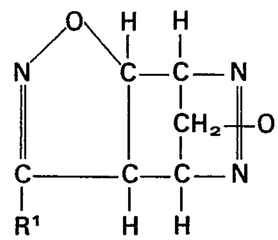

(I)

where $R^1$ has the above meaning, is reacted with an organic peroxide.